# EUROPEAN PATENT SPECIFICATION

(11) **EP 0 806 671 B1**
(45) Date of publication and mention of the grant of the patent: **16.04.2003**
(21) Application number: 96107488.7
(22) Date of filing: 10.05.1996
(51) Int. Cl.: G01N 33/72, G01N 35/02, C12Q 1/28

(54) **Automated urinalysis system for detecting blood in urine**
Automatisches Harnanalysesystem zum Nachweis von Blut im Harn
Système automatisé pour l'analyse d'urine pour détecter du sang dans l'urine

(43) Date of publication of application: 12.11.1997
(73) Proprietor: Chimera Research and Chemical, Inc., Largo, FL 34647 (US)
(72) Inventor: Smith, Jack V., St. Petersburg, FL 33709 (US); Carter, Jesse M., Tempa, FL 33606 (US)
(74) Representative: Tönnies, Jan G.

(56) References cited:
- EP-A- 0 504 663
- US-A- 5 516 700
- PATENT ABSTRACTS OF JAPAN vol. 018, no. 018 (P-1673), 12 January 1994 & JP-A-05 256853 (HITACHI LTD;OTHERS: 01), 8 October 1993,

## Description

### BACKGROUND OF THE INVENTION

This invention relates to a method and materials that are designed for use in automating urinalysis. This system is designed to analyze urine for its constituents by a method that is fully automated (does not require the use of manual methods such as refractometer, pH meter, dipsticks, etc). Automation as designed by this system is directed to the use of a self-operating instrument that is capable of handling multiple reagents designed for use on an automated analyzer system for the quantitative determination of blood in urine.

It is known that the most common method for the analysis of urine is by the use of a manual technique known as a dipstick. This method for the analysis of urine is labor, time intensive, and costly among other detriments. The use of a dipstick for analysis of urine also relies on the subjective interpretation of the technician. The dipstick method requires the technician to submerge the dipstick in a sample of urine and remove it. To wait a specified time, then compare the color development of the test on the dipstick to a color chart. Even more cumbersome methods involve the use of a refractometer, pH meter, or manual chemistry test.

The following list of assay devices utilizing prior art includes dry tablets, dipsticks, or other manual techniques for the analysis urinary constituents. None of the prior devices foresee or teach of a multiple/single liquid reagent system designed specifically for auto-analyzers to quantitatively detecting red blood cells in a patients urine.

One such U.S. Patent No. 4,147,514 discloses test strips (dipsticks) for the detection of ketone bodies. The assay strips are made up of a chemical bonded to a cellulose pad on a strip. This is then dipped into a specimen sample. This method only determines ketone bodies qualitatively at its best, due to inability of the system to allow the use of standards and controls on the same strip the sample is applied to.

Another such patent, U.S. Patent No. 3,146,070 discloses analytical compositions in dry form on a bibulous carrier (dipstick) impregnated with a pH indicator for the determination of pH. This assay at best only determines pH qualitatively, due to the inability to use standards and controls located on the same strip for the same test sample to define and verify a quantitative determination.

Additionally, U.S. Patent No. 4,318,709 discloses a device comprising a carrier matrix (dipstick) impregnated with the test means for specific gravity. This assay at best only determines specific gravity qualitatively, due to the inability to use standards and controls located on the same strip for the same test specimen. The prior art in this case also did not foresee the wide specimen to specimen matrix variations of real world urine samples including matrix components such as pH, and ionic strength, and the concomitant requirement of a multiple reagent system to effectively analyze urine for specific gravity in a liquid to liquid reaction. The normal pH value for urine can range from 4.5 to 8.0, which if using the prior dipstick method the results would be vastly scattered and inaccurate without a reagent to neutralize the effect prior to completion of the assay.

Various devices are described in the literature for the determination of particular urinary constituents one by one with the use of carrier matrices (dipstick, microcapsules, filter pater, etc.). None of the prior art teaches or elucidates a means for detecting by automated technology red blood cells from a single sample of urine, via multiple tests that are reported simultaneously by an autoanalyzer using liquid reagents specifically designed for this family of instruments. As cited by the prior art, (in package insert literature) when evaluating laboratory test results, definitive diagnostic, or therapeutic decisions should not be based on any single result or method. However, the prior art states that dipsticks are affected by substances that cause abnormal urine color, such as drugs oontaining azo dyes (e.g., Pyridium, Azo Gantrisin, Axo, Gantanol), nitrofurantoin (Macrodantin, Furadantin), and riboflavin, and thus may affect the readability of reagent areas on the urinalysis reagent strips (dipsticks). The color development on the reagent pad may be masked, or a color reaction may be produced on the pad that could be interpreted visually and/or instrumentally as a false positive or negative.

Further a state of the art, European Patent document EP-A-0 504 663 (Miles Inc.), describing a peroxidase indicator system for basic media has to be considered. In this document, however, the indicator system is provided to form a chromogen due to the transfer of an electron from the peroxidatively active substance. This system is technically difficult to handle as the coupler and a developer have to be provided which might interfere with the reaction to be tested.

In one further document patent abstract of Japan, Vol. 018, No. 018(P-1673), as well as an JP-A-05256853 a device for diluting a standard sample with a known concentration to an a standard dilution magnification is disclosed. In this document, however, the reagent solutions are not disclosed.

It is an object of the invention therefore to provide for a method for quantitatively detecting red blood cells (RBC) in a patients urine employing an automated analyzer.

### SUMMARY OF THE INVENTION

The automated urinalysis system of this invention offers a method for reducing the consumable materials, and labor costs. The system also offers increased accuracy, sensitivity, and objective quantifiable determinations of urinary constituents for better diagnostic interpretation of the test results of urine, thus enabling a physician to provide better health care for the patient.

This invention satisfied many of the problems unanswered by the prior art: quantitative results, non-subjective results, reproducible results, increased accuracy, precision, sensitivity, carrier free reagents, reagents designed for autoanalyzer use, reagents uniquely designed for each particular urine analyte assay overcoming matrix problems previously unanswered by prior art, a method allowing vast improvement of test completion time (hundreds to thousands per hour). The present invention presents a fully automateable walk-away urinalysis system applicable to any discrete autoanalyzer currently in use, and obviously represents a marked advancement in the art of urinalysis. The clear cut object of the present invention is to provide a more comprehensive method for determining urinary constituents (Leukocytes, Blood, Bacterial Nitrite/Indole/reductase activity, Total Ketone bodies, Glucose, Protein, pH, and specific gravity) that in general benefit society as a whole and specifically yield improved health care.

### DETAILED DESCRIPTION OF THE INVENTION

The presently claimed method comprises a group of carrier-free liquid reagents designed for simultaneous usage on automated analyzers for quantitative determination of urinary constituents. The automated urinalysis system of the instant invention solves the problems confronting automating the analysis of urine, and in the process, represents a significant improvement over the present art. These improvements which facilitate application to automation and represent significant technical improvement over the previous art include, a buffering system for pH variation in urine by correcting pH to the analytically preferred value prior to analysis and also stabilizing reaction rates thereby improving linearity and neutralizing the interference effects of the highly complex matrix of random urines submitted for analysis. Additional technical improvement is due to the addition of components to remove interfering substances yielding reduced assay limitations and increased linearity, accuracy and precision in the resulting quantitations. These unique reagent formulations allow automation resulting in, but not limited to, enhanced speed, objectivity, accuracy and sensitivity associated with the automated test. A synopsis of the automated testing process follows. The entire automated urinalysis reagent system is loaded into an autoanalyzer. The controls, standards and unknown urine samples are fed into the autoanalyzer, individually mixed with the test reagent in discrete cuvettes, the absorbance read and quantitation determined for comparison with the standard curve.

The composition of the reagent of the present invention is designed for optimum reaction with the random urine samples and to effectively deal with problems arising from the tremendous variability from sample to sample due to the diet, disease state, medications, time of collection, state of hydration, sex, age and physical well being of the patient. All of the factors can interfere with the previous art.

The automated urinalysis system reagents are individually designed for optimum analysis of the specific urinary component. The reagent system to detect Blood (RBC's) in urine is carrier independent and contains specific agents added to compensate for interference by urea, vitamin C, high ionic levels (specific gravity), abnormal pH and other normal urinary constituents. The RBC reagent system is composed of one reagent one). The first reagent (R1) is specifically designed to neutralize matrix interference and increase sample-reagent compatibility, with the autoanalyzer. 2, 3-Butanedione monoxime is added to the first reagent (R1) to remove urea and other substances in the urine sample that cause interference with colorimetric reactions utilizing any of the following components: 3,3'5,5'-Tetramethylbenzidine, Dicarboxidine, 3-Methyl-2-benzothiazolinone hydrazone, or N, N- dimethylaniline. The components listed above are particularly susceptible to interference from urea (a major component of urine). Ethylenediaminetetraacetic acid (disodium salt), 0.15 M succinic acid and dimercaptopropanol are other oomponents of the R1 used to neutralize interfering substances by chelation and anti-oxidant activity. This compound removes oxidizing contaminants such hypochlorite and acts as a solution clarifier. It causes the disappearance of the characteristic yellow color of urine, thereby enhancing spectrophotometric analysis. 2,3-Diphosphoglycerate is added to affect the oxygen disassociation of hemoglobin. Saponin or 25% acetic acid is present to lyse the red blood cells that may be present and intact in urine, thus releasing the hemoglobin contained within. Note that 2,3-Diphosphoglycerate in the alkaline reagent mixture causes the disassociation constant of hemoglobin to shift to the left (acid Bohr effect), thus increasing the affinity of hemoglobin for oxygen and forcing the reaction to completion. Oxygen is provided by the reaction of hemoglobin with hydrogen peroxide. Sodium azide is added to stabilize hydrogen peroxide. The R2 contains a 30% solution of hydrogen peroxide acting as a substrate for the peroxidase activity of the heme fraction of hemoglobin which is a major component of red blood cells. The R1 also contains a buffer to adjust sample pH and aid in solubility and compatibility with R1's complex chemical matrix. This complex reagent matrix requires a complementary buffering system with unique dynamics, capable of adjusting the reaction solution to the ideal pKa and promoting component solution compatibility of the aqueous medium with autoanalyzers. Unbuffered solutions may have high acidic or basic activity, or strictly organic properties which are not compatible with autoanalyzer syringes, tubing, metal and plastic parts. This reagent system buffer is designed to correct these problems. The buffers also promote carrier independence. The R1 also contains surfactants that decrease surface tension, promote effective mixing on a molecular level and improve flow dynamics through tubing and syringes of automated analyzers. The concentrations of R1 buffers and components can be varied to compensate for limitations and variations in the configuration of sampling and reagent delivery systems of various makes of autoanalyzers. The R1 components compensate for abnormal urinary pH and highly buffered urines. Ampyrone is added to the R1 to promote, or catalyze the reaction of the aforementioned oxidized peroxide molecule with the coupling agent

2, 2'-Azino-di-3-ethylbenzthiazoline sulfonic acid diammonium salt (ABTS).

The addition of Pyrogallol is added to R1 and acts as a substrate that is oxidized by the oxygen radical released when the heme (peroxidase active) molecule reacts with hydrogen peroxide in solution.

The second reagent (R2) of the two part reagent system for Blood (if a single reagent system for Blood is not used) is composed of one, or more of the following: 3,3'5,5'-tetramethylbenzidine, dicarboxidine, pyrogallol, hydrogen peroxide, 3-methyl-2-benzothiazone hydrazone, N,N-dimethylaniline, benzidine, o-dianisidine and oxidized phenothiazines in solution. This reagent is buffered according to which group or single component is used. This buffer contained in R2 adjusts sample pH and aids in solubility and compatibility of R2's complex chemical matrix. This complex reagent matrix requires a complementary buffering system with unique dynamics capable of adjusting the reaction solution to the ideal pKa's, establishing carrier independence and promoting component solution compatibility in an aqueous medium with autoanalyzers. Unbuffered solutions may have high acidic or basic activity or strictly organic solubilities properties which are not compatible with autoanalyzer syringes, tubing, metal and plastic parts. The R2 also contains a color indicator such as 2,2' azinobis (3-ethylbenzothiazoline)-6-sulfonic acid and surfactants that decrease surface tension, promote effective mixing on a molecular level, enhance carrier independence and improve flow dynamics through tubing and syringes of automated analyzers. The combinations and concentrations of R1 and or the R2 components can be varied due to limitations and variations in the configuration of sampling and reagent delivery systems of different makes of autoanalyzers.

The preprogrammed monochromatically specified wavelength employed depends on the autoanalyzer employed, but will generally range from 340 to 700 nanometers.

Without further elaboration, it is believed that one skilled in the art can, using the preceding description, effectively utilize the present invention. The following preferred specific embodiments are, therefore, to be merely illustrative and not limitive of the remainder of the disclosure of the present invention in any way whatsoever. In the following examples, all instrument parameters, reagent combinations and method techniques are set forth.

### EXAMPLE 1

The following examples do not fall within the scope of the claims and are therefore included for comparative purposes:

The automated RBC urinalysis reagent system's first reagent (R1) contains surfactant, 2, 3-Butanedione monoxime, ethylenediaminetetraacetic acid, dimercaptopropanol, saponin, 2,3-Diphosphoglycerate and buffer. The second reagent (R2) consists besides a compound to lyse and a substrate to react with the RBC of surfactant, buffer, 3,3',5,5'-tetramethylbenzidine in 10% lactic acid. These reagents are placed in the autoanalyzer. The urine samples, standards and controls are placed in the autoanalyzer specimen cups. The urine samples, standards and controls are aliquoted into cuvettes, mixed with the first reagent and then mixed with the second reagent and then read at specified intervals as dictated by the instrument parameters and at the specified wavelengths (monochromatically) depending on reagent combination used. In this instance, the assay should be read at 660 nanometers with read times specific to the analyzer.

### EXAMPLE 2

The automated RBC urinalysis single reagent system would contain (all or some of the following:) 2,3-butanedione monoxime, ethylenediaminetetraacetic acid, dimercaptopropanol, 2,3-diphosphoglycerate, Ampyrone, sodium azide, hydrogen peroxide, saponin, p-hydroxybenzoic acid, N-ethyl-N-sulfohydroxypropyl-m-toluidine. The reagents are placed on the autoanalyzer. The urine samples, standards and controls are placed in the autoanalyzer specimen cups. The urine samples, standards and controls are aliquoted into cuvettes, mixed with the reagent and the solutions are read at specified intervals as dictated by the instrument parameters and the specified wavelength (monochromatically) depending on the reagent combination used. In this instance, the assay should be read at 505 nanometers read times are specific to the analyzer.

### EXAMPLE 3

In the automated RBC urinalysis reagent system, first reagent (R1), contains surfactants, buffer, saponin, and etheylenediaminetetraacetic acid. The second reagent (R2) consists of hydrogen peroxide, 3-methyl-2-benzothiazoline hydrazone, N,N-dimethylanilane, buffers and surfactants. The reagents are placed on the autoanalyzer. The urine samples, standards and controls are placed in the autoanalyzer specimen cups. The urine samples, standards and controls are aliquoted into cuvettes with the first reagent. The second reagent is then added and mixed and the solutions are then read at specified intervals as dictated by the instrument parameters at the specified wavelength (monochromatically) depending on the reagent combination used. In this instance, the assay should be read at 585 nanometers and read times are specific to the analyzer.

### EXAMPLE 4

In the automated RBC urinalysis reagent system's first reagent (R1) contains a buffer. The second reagent R2 consists besides a compound to lyse and a substrate to react with the RBC of buffer, o-dianisidine. The reagents are placed on the autoanalyzer. The urine sample, standards and controls are placed in the autoanalyzer specimen cups. The urine samples, standards and controls are aliquoted into cuvettes, mixed with the first reagent, the second reagent is then added and mixed and the solutions are then read at specified intervals as dictated by the instrument parameters at the specified wavelength (monochromatically) depending on the reagent combination used. In this instance, the assay should be read at 540 nanometers and read times are specific to the analyzer.

### EXAMPLE 5

In the automated RBC urinalysis the single reagent system would contain all or some of the following: 2,3-butanedione monoxime, pyrogallol, ethylenediaminetetraacetic acid, p-hydroxybenzoic acid, hydrogen peroxide, N-ethyl-N-(2-hydroxy-3-sulfopropyl)-m-toluidine and surfactants. The reagents are placed on the autoanalyzer. The urine samples, standards and controls are placed in the autoanalyzer specimen cups. The urine samples, standards and controls are aliquoted into cuvettes, mixed with the reagent, and the solutions are read at specified intervals as dictated by the instrument parameters at the specified wavelength (monochromatically) depending on reagent combination used. In this instance, the assay should be read at 550 and read time is specific to the analyzer.

### EXAMPLE 6

The automated RBC urinalysis reagent system's first reagent (R1) contains ethylenediaminetetraacetic acid and buffer. The second reagent R2 consist besides a compound to lyse and a substrate to react with the RBC of buffer and oxidized phenothiazines. The reagents are placed on the autoanalyzer. The urine samples, standards and controls are placed in the autoanalyzer specimen cups. The urine samples, standards and controls are aliquoted into cuvettes, mixed with the first reagent, then the second reagent is then added and mixed and the solutions are then read at specified intervals as dictated by the instrument parameters at the specified wavelength (monochromatically) depending on the reagent combination used. In this instance, the assay should be read at 540 nanometers and read times are specific to the analyzer.

### EXAMPLE 7

In the automated RBC urinalysis reagent system's first reagent (R1) contains surfactant, 2,3-diphosphoglycerate, hydrogen peroxide, and buffer. The second reagent R2 consist of surfactant, buffer, p-hydroxybenzoic acid and phenol. The reagents are placed on the autoanalyzer. The urine samples, standards and controls are placed in the autoanalyzer specimen cups. The urine samples, standards and control are aliquoted into cuvettes and mixed with the first reagent. The second reagent is then added and mixed and the solutions are then read at specified intervals as dictated by the instrument parameters at the specified wavelength (monochromatically) depending on the reagent combination used. In this instance, the assay should be read at 505 nanometers and read times are specific to the analyzer.

### EXAMPLE 8

The automated RBC urinalysis reagent system's first reagent (R1) contains ethylenediaminetetraacetic acid, 2,3-diphosphoglycerate, hydrogen peroxide, surfactants and buffers. The second reagent (R2) has buffers, surfactants, N-ethyl-N-sulfohydroxypropyl-m-toluidine. The reagents are placed on the autoanalyzer. The urine samples, standards and control are aliquoted into cuvettes, mixed with the first reagent, the second reagent is then added and mixed and the solutions are then read at specified intervals as dictated by the instrument parameters at the specified wavelength (monochromatically) depending on the reagent combination used. In this instance, the assay should be read at 550 nanometers and read times are specific to the analyzer.

### EXAMPLE 9

The automated RBC urinalysis reagent system's first reagent (R1) contains hydrogen peroxide, surfactants and buffers. The second reagent (R2) consists of buffers, surfactants, N-Ethyl-N-sulfohydroxypropyl-m-toluidine (TOOS) and/or (one or more from the following group: 2,2'azino-di-(3-ethylbenzthiazoline) sulfonic diammonium salt (ABTS), 2-hydroxy-3,5-dichlorobenzenesulfonate sodium salt (HDCBS) or other suitable trinder reagent. The reagents are placed on the autoanalyzer. The urine samples, standards and control are aliquoted into cuvettes and mixed with the first reagent. The second reagent is then added and mixed and the solutions are then read at specified intervals as dictated by the instrument parameters at the specified wavelength (monoohromatically) depending on the reagent combination used. In this instance, the assay should be read at 550 nanometers and read times are specific to the analyzer.

## Claims

1. Method for quantitatively detecting red blood cells in a patient's urine employing an automated analyzer with the method steps:
- placing an aliquot of the urine to be tested in an automated analyzer sampling cup,
- placing the cup in a sampling tray within the automated analyzer,
- transferring the urine to a cuvette mounted within the automated analyzer,
- injecting at least on reagent composition in an aqueous medium into the cuvette,
the reagent composition containing :
-- a buffer to adjust the pH of the urine to a preferred value,
-- a compound to lyse the red blood cells in the urine,
-- a substrate to react with the red blood cells in the urine, and
-- a single color indicator to quantitatively determine blood cells in the urine,
wherein said indicator is 2,2'-Azino-bis (3-ethylbenzthiazoline-6-Sulfonic Acid),
- reading at specified intervals in accordance with a preprogrammed code introduced into the automated analyzer, at a preprogrammed monochromatically specified wavelenght, to compare absorbance of the patient's urine and reagent composition complex with that of a standard containing a known concentration of hemoglobin and thereby determining the presence or absence of red blood cells in the patient's urine.

2. Method according to claim 1, **characterized in that** the reagent composition is injected into the cuvette by means of first and second compositions, a first composition containing at least one compound to neutralize urine pH interference and lyse the red blood cells and the single color indicator and a second reagent composition containing the substrate.

3. Method according to one of the proceeding claims, wherein the wavelength of the analyzer is about 340 to 700 nanometers.

4. Method according to one of the proceeding claims, wherein the at least one compound to neutralize urine pH interference and lyse red blood cells is glacial acetic acid.

5. Method according to one of the proceeding claims, wherein the second reagent substrate is hydrogen peroxid.

## Patentansprüche

1. Verfahren zum quantitativen Erfassen roter Blutzellen im Urin eines Patienten unter Nutzung eines automatisierten Analysiergerätes mit den Verfahrensschritten:
- Einbringen eines Aliquots des zu testenden Urins in eine Probenschale des automatisierten Analysiergerätes,
- Einbringen der Probenschale in ein Probentablett innerhalb des automatisierten Analysiergerätes,
- Überführen des Urins in eine Küvette, die innerhalb des automatisierten Analysiergerätes befestigt ist,
- Injizieren wenigstens einer Reagenzien-Zusammensetzung in einem wässrigen Medium in die Küvette,
wobei die Reagenzien-Zusammensetzung enthält:
-- einen Puffer, um den pH des Urins auf einen bevorzugten Wert einzustellen,
-- eine Zusammensetzung, um die roten Blutzellen in dem Urin in Lysis zu bringen,
-- ein Substrat, um mit den roten Blutzellen in dem Urin zu reagieren, und
-- einen einzelnen Farbindikator, um quantitativ die Blutzellen in dem Urin zu bestimmen,
wobei der Indikator 2,2'-Azino-bis (3-Ethylbenzthiazolin-6-sulfonische Säure) ist,
- Lesen an bestimmten Intervallen nach einem vorbestimmten Code, der in das automatisierte Analysiergerät eingegeben ist, an einer vorprogrammierten monochromatischen bestimmten Wellenlänge zum Vergleich der Absorption des Patientenurins und des Komplexes der Reagenzien-Zusammensetzung mit der eines Standards, der eine bekannte Konzentration von Hämoglobin enthält, und hierdurch Bestimmen des Vorhandenseins oder Fehlens von roten Blutzellen im Urin des Patienten.

2. Verfahren nach Anspruch 1, **dadurch gekennzeichnet, dass** die Reagenzien-Zusammensetzung in eine Küvette mittels erster und zweiter Zusammensetzungen injiziert wird, wobei eine erste Zusammensetzung wenigstens eine Verbindung umfasst, um die Urin-pH-Interferenz zu neutralisieren und die roten Blutzellen in Lysis zu bringen, und der einzelne Farbindikator und eine zweite Reagenzien-Zusammensetzung das Substrat enthält.

3. Verfahren nach einem der vorangehenden Ansprüche, **dadurch gekennzeichnet, dass** die Wellenlänge des Analysiergerätes ca. 340 bis 700 Nanometer beträgt.

4. Verfahren nach einem der vorangehenden Ansprüche, **dadurch gekennzeichnet, dass** wenigstens eine Verbindung zur Neutralisierung der Urin-pH-Interferenz und dem In-Lysis-Bringen der roten Blutzellen Eis-Essig ist.

5. Verfahren nach einem der vorangehenden Ansprüche, **dadurch gekennzeichnet, dass** das Substrat der zweiten Reagenz Wasserstoffsuperoxyd ist.

## Revendications

1. Procédé de détection quantitative des globules rouges dans l'urine d'un patient à l'aide d'un analyseur automatisé comprenant les phases suivantes :
- disposition d'un aliquote de l'urine à éprouver dans une coupelle d'échantillonnage d'analyseur automatisé,
- disposition de la coupelle dans un plateau d'échantillonnage à l'intérieur de l'analyseur automatisé,
- transfert de l'urine dans une cuvette montée à l'intérieur de l'analyseur automatisé,
- injection d'au moins une composition réactive dans un milieu aqueux dans la cuvette,
la composition réactive contenant :
-- un tampon permettant d'ajuster l'acidité pH de l'urine à une valeur de prédilection,
-- un composé permettant de lyser les globules rouges dans l'urine,
-- un substrat réactif aux globules rouges dans l'urine, et
-- un témoin monochrome permettant de déterminer quantitativement les globules rouges dans l'urine,
où ledit témoin est du 2,2'-Azino-bis (acide 3-éthylbenzthiazoline-6-sulfonique),
- lecture à des intervalles spécifiés selon un code préprogrammé introduit dans l'analyseur automatisé, à une longueur d'onde spécifiée préprogrammée et de manière monochromatique, pour comparer l'absorbance de l'urine du patient et du complexe composé réactif avec une norme contenant une concentration connue d'hémoglobine et déterminant ainsi la présence ou l'absence de globules rouges dans l'urine du patient.

2. Procédé selon la revendication 1, **caractérisé en ce que** la composition réactive est injectée dans la cuvette au moyen d'une première et d'une deuxième compositions, une première composition contenant au moins un composé pour neutraliser l'interférence pH de l'urine puis lyser les globules rouges et le témoin monochrome, et une deuxième composition réactive contenant le substrat.

3. Procédé selon l'une de revendications précédentes, où la longueur d'onde de l'analyseur est de l'ordre de 340 à 700 nanomètres.

4. Procédé selon l'une des revendications précédentes, où au moins un composé permettant de neutraliser l'interférence pH de l'urine et de lyser les globules rouges, est de l'acide acétique glacial.

5. Procédé selon l'une des revendications précédentes, où le deuxième substrat réactif est du peroxyde d'hydrogène.
